**Europäisches Patentamt**

**European Patent Office**

**Office européen des brevets**

(19)

(11) Veröffentlichungsnummer: **0 150 436 B1**

(12) ## EUROPÄISCHE PATENTSCHRIFT

(45) Veröffentlichungstag der Patentschrift:
20.11.86

(51) Int. Cl.⁴: **C 07 C  47/04,** C 07 C  45/38, C 07 C  45/78, C 07 C  45/81, C 07 C  45/80

(21) Anmeldenummer: **84115710.0**

(22) Anmeldetag: **18.12.84**

(54) **Verfahren zur Herstellung von Formaldehyd.**

(30) Priorität: **28.01.84  DE 3402995**

(43) Veröffentlichungstag der Anmeldung:
**07.08.85 Patentblatt 85/32**

(45) Bekanntmachung des Hinweises auf die Patenterteilung:
**20.11.86 Patentblatt 86/47**

(84) Benannte Vertragsstaaten:
**BE CH DE FR GB IT LI NL**

(56) Entgegenhaltungen:
**GB - A - 577 573**

(73) Patentinhaber: **BASF Aktiengesellschaft,
Carl-Bosch-Strasse 38, D-6700 Ludwigshafen (DE)**

(72) Erfinder: **Aicher, Albrecht, Sonnenstrasse 21,
6710 Frankenthal (DE)**
Erfinder: **Lehmann, Gunter, Schlesier Strasse 4,
6701 Birkenheide (DE)**
Erfinder: **Petri, Norbert, Dr., Max-Beckmann-Strasse 17,
6710 Frankenthal (DE)**
Erfinder: **Pitteroff, Walter, Dr., In den Hahndornen 10,
6719 Bobenheim (DE)**
Erfinder: **Reuss, Guenter, Dr., Theaterplatz 10,
6700 Ludwigshafen (DE)**
Erfinder: **Schreiber, Hans, Osloer Weg 35,
6700 Ludwigshafen (DE)**
Erfinder: **Sebastian, Robert, Katharinenstrasse 4,
6705 Deidesheim (DE)**

## Beschreibung

Die Erfindung betrifft ein Verfahren zur Herstellung von Formaldehyd durch a) Einleitung von Methanol und Wasser mit einer Konzentration zwischen 50 und 90 Gew.% Methanol, bezogen auf das Gesamtgewicht der beiden Stoffe, in eine Füllkörperkolonne, die Füllkörper mit einer Gesamtschichtdicke von mindestens 50 cm und einer Gesamtoberfläche von mindestens 0,5 cm² je cm³ Füllkörperschicht, einen Kreislauf an Flüssigkeit von 15 bis 90 g/min je g/min der Kolonne zugeführtes Methanol, eine Konzentration der Kreislaufflüssigkeit von 10 bis 45 Gew.% Methanol, eine Temperatur am Eingang der Kreislaufflüssigkeit in die Kolonne von 50 bis 86°C, und eine von dieser Eingangstemperatur um 10 bis 25°C niedrigere Sumpftemperatur von 40 bis 70°C besitzt, b) Abtrennung eines Anteils an Methanol und Wasser aus der Kolonne durch Strippen mit Luft, Inertgas und/oder Abgas mit einem Durchsatz von 0,5 bis 3 t Methanol und Wasser pro Stunde und Quadratmeter Kolonnenquerschnitt, c) Umsetzung des gasförmigen Gemischs von Methanol, Wasser sowie Luft, Inertgas und/oder Abgas mit einer Belastung von 0,5 bis 3 t Methanol je m² Katalysatorbettquerschnitt und Stunde in Gegenwart eines Silberkatalysators bei einer Temperatur von 550 bis 750°C und d) schliesslich Abkühlung und Absorption der heissen Reaktionsgase und Verwendung der anfallenden Absorptionswärme und gewünschtenfalls Reaktionswärme und/oder Kondensationswärme ganz oder teilweise zur Erhitzung der Kreislaufflüssigkeit, wobei gewünschtenfalls Verunreinigungen enthaltendes Methanol verwendet wird und nach den Stufen a) und b) und vor den Stufen c) und d) die Massnahmen einer Stufe e) durchgeführt wird, worin man das gebildete gasförmige Gemisch durch zwei Schichten Drahtfilter mit einem Drahtdurchmesser von 0,1 bis 0,5 mm und einem freien Schichtvolumen von 90 bis 99,5 Vol.%, bezogen auf das Gesamtvolumen einer Drahtschicht, mit einer Strömungsgeschwindigkeit von 7 bis 13 m/sec in der ersten Schicht und von 1 bis 4 m/sec in der zweiten Schicht, leitet.

Es ist aus Ullmann, Encyklopädie der technischen Chemie (4. Aufl.) 11, S. 692 bekannt, eine 60 gew.%ige wässerige Methanollösung zu verdampfen, mit Luft zu vermischen und in Gegenwart von Silberkatalysatoren umzusetzen. Wie aus BIOS, Final Report, Nr. 978, Item No. 22, Seite 43, bekannt ist, kann man die Luft auch durch den Sumpf des Verdampfers, der auf 87°C gehalten wird, führen und so das Ausgangsgemisch der Umsetzung herstellen. Im Verdampfer, der von aussen durch Dampf beheizt wird, werden 55%ige, wässerige Methanollösungen verdampft. Diese Verfahren werden mit reinem Methanol durchgeführt.

Rohmethanol kann je nach Herstellungsverfahren (Ullmanns Encyklopädie der technischen Chemie (3. Aufl.), Band 12, Seiten 398 ff.) in seiner Zusammensetzung variieren und enthält im allgemeinen von 95 bis 70 Gew.% Methanol, von 1 bis 29,9 Gew.% Wasser und von 0,1 bis 6 Gew.% Verunreinigungen. Als Verunreinigungen kommen je nach Herstellung und Lagerung z.B. Alkalisalze wie Natriumformiat, Natriumhydrogencarbonat, Natriumcarbonat, Natriumacetat, Natriumsulfid; Natrium bzw. Natriummethylat, Kaliumhydroxid, Natriumhydroxid; Ameisensäure; Aldehyde wie Acrolein, Glyoxal, Butyraldehyd, Propionaldehyd, Acetaldehyd; Ketone wie Aceton und Butanon-2; Glykol, Diglykol, Triglykol, und höhere Alkanole wie N-Butanol, Isobutanol, Isopropanol, n-Propanol, n-Pentanol, Isohexanol, Isoheptanol, n-Hexanol; Ether wie Glykol-, Diglykol-methylether, Dimethylether; aliphatische, cycloaliphatische, aromatische Kohlenwasserstoffe wie Benzol, Toluol, Xylol, Dekan, Undekan, Dodekan, Cyclohexan, Ethylbenzol; organische oder anorganische Verbindungen, z.B. Formiate, Chloride und Sulfide von Metallen wie Eisen, Chrom, Kupfer, Aluminium, Zink, Magnesium; Schwefelverbindungen wie Dimethylsulfid; Ester wie z.B. Dimethylterephthalat; Amine wie Monomethylamin, Dimethylamin, Trimethylamin; Ammoniak in Frage. Insbesondere sind alkalische Verunreinigungen in der Regel vorhanden, da die im Methanol vorhandene Säure bei fast allen Syntheseverfahren mit Alkali neutralisiert wird.

Beim Verdampfendes Rohmethanols nach den üblichen Methoden gelangen nicht nur dampfförmige, sondern auch flüssige und feste Verunreinigungen in das dampfförmige Ausgangsgemisch der Formaldehydsynthese, beispielsweise in Gestalt von feinverteilten Tröpfchen oder Feststoffen bzw. Flüssigkeitsnebeln. Sie begünstigen während der Umsetzung des Methanols Nebenreaktionen bzw. greifen den Katalysator an, z.B. durch Zerstörung der aktiven Oberfläche des Silbers oder durch Ablagerung von Feststoffen oder harzartigen Körpern auf dem Katalysator, und setzen seine Lebensdauer und damit die Ausbeute an Endstoff und die Wirtschaftlichkeit des Verfahrens herab. Daneben können Ablagerungen durch Verstopfung der Zuleitungen oder Korrosion an den Metallflächen den Betrieb einer Anlage wesentlich stören. Der Katalysator, der aus einem Granulat von Silberkörnern besteht, verliert durch die Ablagerungen nach und nach seine Durchlässigkeit für Gase. Der Druckverlust an der Katalysatorschicht steigt und bedingt einen höheren Energieaufwand für die Luftkompression. Mit den im allgemeinen üblichen Gebläsen kann nicht mehr die notwendige Menge an Luft durchgeleitet werden und der Umsatz der Anlage sinkt, was zu vorzeitigem Abschalten der Anlage zwingt, um den Katalysator zu erneuern. Hierin liegt ein weiterer Grund für Ausbeuteverluste. Ausserdem bedeutet kürzere Lebensdauer des Katalysators Mehrausgaben für Wechsel und Regenerierung des Katalysators.

Eine weitere Ursache für eine Katalysatorvergiftung liegt in der Wirkung schädlicher Fremdsubstanzen, die in der für die Oxidation verwendeten Luft enthalten sind. Solche Luftverunreinigungen treten in besonderem Masse in der Nähe von industriellen Ballungsgebieten auf und enthalten z.B. folgende, den Katalysator vergiftende Komponenten: Schwefelwasserstoff, Schwefeldioxid, Chlorwasserstoff, Fluorwasserstoff, Halogen, flüchtige Halogenverbindungen wie Tetrachlorkohlenstoff; Ammoniak, Amine wie Monomethylamin, Dimethylamin, Trimethylamin; Arsen- und Antimonverbindungen wie Arsentrioxid, Antimontrioxid; Acetylen, Phosphorverbindungen wie Phosphorwasserstoff, Russ, Eisenoxidstaub, Cyanwasserstoff, Kohlenmonoxid; aus der anaeroben Zersetzung eiweisshaltiger Abfallstoffe entstehende Fremdstoffe wie Mercaptane, Indol, Skatol; Stickstoffoxide; Bleiverbindungen wie Tetraethyl- und Tetramethylblei; organische Verbindungen wie 3,4-Benzpyren, Fluoranthren, Pyren, Phenanthren, die durch Autoabgase in die Luft gelangen, und deren Oxidationsprodukte wie Acrolein. Im allgemeinen beträgt der Fremdstoffanteil in der Luft 0,01 bis 10 ppm.

Als Wasser wird bei der Formaldehydsynthese Kondenswasser, aber auch chlorfreies, zweckmässig enthärtetes Betriebswasser verwendet. Als Betriebswasser in diesem Zusammenhang kommen Grundwasser, Quellwasser, Oberflächenwasser wie Flusswasser, Trinkwasser, Kesselspeisewasser und gelegentlich auch Meerwasser in Betracht. Je nach Herkunft und Aufbereitung kann das verwendete Wasser zahlreiche Stoffe als Verunreinigungen enthalten, z.B. Metallsalze wie Mangansulfat, Eisenchlorid, Erdalkaliverbindungen in Gestalt der Wasserhärte, Ammonium-, Alkalisalze, Metalle wie Zink oder Aluminium oder Kupfer z.B. aus Rohmaterialien, Nitrate, Silikate, Nitrite, Fluoride, Phosphate, organische Zersetzungsprodukte wie Phenole.

Bei der Verdampfung solcher Gemische von Methanol und Wasser treten, häufig in erheblichem Masse, Schwierigkeiten auf: Die Verdampfungsgeschwindigkeit nimmt ab, der Verdampfersumpf hält Flüssigkeit zurück und es treten an der Oberfläche der verdampfenden Methanollösung grössere Mengen an Schaum mit relativ grossen Einzelblasen und meist relativ fester Konsistenz auf. Gleichzeitig kann der Druck im Verdampfer zunehmen und die Flüssigkeit unter Vermischung mit der Luft mit der Zeit in wachsendem Masse eine dichte Schaumschicht ausbilden. In manchen Fällen wird der Schaum mitgerissen, gelangt auf den Katalysator und stört bzw. verhindert die Umsetzung des Methanols. Gerade im grosstechnischen Betrieb, wo im allgemeinen aus 60- bis 95-gewichtsprozentigen Methanollösungen Mengen von 1 000 bis 20 000 kg Methanollösung pro Stunde verdampft werden, kann nach 1 bis 16, häufig schon in Abständen von 1 bis 3 Stunden, der Druck von üblicherweise 1,2 bar auf 1,5 bis 1,8 bar ansteigen, wenn eine zweibödige Verdampferkolonne verwendet wird. Gleichzeitig nimmt die Menge an verdampfter Lösung je Stunde auf 70 bis 80% ihres ursprünglichen Wertes ab. Alle diese Schwierigkeiten führen zu meist erheblichen Betriebsstörungen bzw. Betriebsausfällen. Zumindest sind Betriebsunterbrechungen in bezug auf Zulauf und Wärmezuführung notwendig.

Daneben ergeben sich bei den bekannten Verfahren betriebliche Schwierigkeiten bei Inbetriebnahme der Verdampfungsanlagen, gerade auch nach Betriebsstörungen bzw. Stillstand der Anlagen. Luft steht in solchen Fällen rasch zur Verfügung, da die Gebläse sehr schnell volle Leistung erbringen. Jedoch kostet das Anheizen des Verdampfers wertvolle Zeit. Man benötigt deshalb grössere Flüssigkeitsmengen auf jedem Boden als Wärmespeicher, so dass die Wärme für eine rasch notwendige Verdampfung zur Verfügung steht. Glockenbodenkolonnen und Füllkörperkolonnen entleeren sich aber rasch. Auch Siebbodenkolonnen herkömmlicher Bauart helfen hier wenig, sie sind empfindlich gegen Belastungsschwankungen und brauchen ebenfalls längere Zeit zur Aufheizung; daneben neigen sie eher zu Schwierigkeiten beim Durchtritt des Dampfes durch die Sieblöcher, da der Dampf die auf den Böden sich ansammelnde Flüssigkeit in Gestalt von Tropfen mitreisst bzw. häufig die Flüssigkeit die Löcher verstopft und «durchregnet».

Ventilbodenkolonnen sind teuer und bieten einen hohen Widerstand bei hoher Belastung. Ausserdem schliessen die Ventile nach längerer Betriebszeit nicht völlig dicht, so dass auch diese Kolonnen sich rasch entleeren. Hinzu kommt, dass bei den genannten Herstellungsverfahren die Belastung besonders hoch ist, da das Dampfgemisch und Luft als Ausgangsgemisch der Formaldehydherstellung dienen. Wird eine Anlage in Betrieb genommen, so perlt aus vorgenannten Gründen das Gasgemisch von Luft und Dampf nur sehr träge durch die Flüssigkeit des Verdampfungsraumes. Der Dampf tritt nicht mehr oder nicht genügend in Austausch mit der Flüssigkeit, da die Durchmischung gering ist. Die Folge ist eine starke Anreicherung von Methanol im Verdampfersumpf und somit eine Belastung des Abwassers, was Umweltprobleme aufwirft und die Wirtschaftlichkeit des Verfahrens herabsetzt. Schliesslich muss zu jedem Zeitpunkt, auch bei niedriger Belastung, sichergestellt sein, dass eine ausreichende Menge an Methanol beim Durchleiten der Luft verdampft; andernfalls entstehen luftreiche Mischungen, die explosionsfähig sind. Jeder Stillstand der Anlage bedingt somit aus den genannten Gründen besondere Operationen zur Inbetriebnahme.

Die DE-OS 23 23 758 beschreibt verschiedene ältere technische Verfahren sowie einbezüglich vorgenannter Schwierigkeiten vorteilhafteres Verfahren. Man verfährt in der Weise, dass man in einer Bondenkolonne mit mehreren Glockensiebböden, von denen jeder einen oberen, nach aussen zu abfallenden Siebboden mit einem Lochdurchmesser von 2 bis 15 mm und einen unteren, nach aussen zu ansteigenden, tellerförmigen Boden besitzt und der Siebboden einen Winkel von 2 bis 10° und der untere Boden einen Winkel von 4 bis 20° mit der Waagrechten bildet, Methanol und Wasser unter Vermischung mit Luft verdampft, wobei die flüssigen Stoffe am Kopf und die Luft am Fuss der Kolonne zugeführt werden, und das so erhaltene dampfförmige Ausgangsgemisch oxidierend dehydriert. Doch auch dieses Verfahren zeigt Nachteile; die Siebböden sind in ihrem Betrieb kompliziert und in der Fertigung aufwendig.

Die DE-OS 21 14 370 zeigt, dass die Apparate zur Verdampfung von Methanol im allgemeinen mit Tröpfchenabscheidern ausgerüstet werden (BIOS Final Report No. 1331; FIAT Final Report No. 999), z.B. Raschigringpackungen oder Pakkungen aus Drahtgeweben. Solche Packungen haben einen gewissen Effekt, bis sie genügend Flüssigkeit enthalten. Von da an schiebt sich ein Teil der Flüssigkeit hindurch und versprüht hinter der Packung wieder zu Tröpfchen oder Nebeln. Eine weitgehende Belagsbildung auf dem Katalysator kann somit auch durch diese Massnahmen nicht verhindert werden. Die DE-OS 21 14 370 weist daher darauf hin, dass aus den genannten Gründen eine grosstechnische Verwendung von Rohmethanol für die Formaldehydherstellung nicht gegeben war. Es werden weitere bekannte technische Massnahmen bei der Verwendung von Rohmethanol, z.B. Destillation, Behandlung des Methanols mit Alkalien und/oder Oxidationsmitteln, oder Herstellung von Formaldehyd durch ein Niederdruckverfahren geschildert und die Nachteile der entsprechenden Verfahren aufgeführt.

Die DE-OS 21 14 370 empfiehlt ein zu diesem Stand der Technik vorteilhafteres Verfahren mit Rohmethanol als Ausgangsstoff, wobei man die Umsetzung mit Rohmethanol, das in Dampfform vor Eintritt in die Katalysatorzone gegen Prallwände geleitet wird und die dabei an den Prallwänden in fester oder flüssiger Form anfallenden Anteile an Verunreinigungen abgetrennt werden, durchführt. Der Dampf/Gasstrom, der gegen die Prallwände geleitet wird, hat zweckmässig eine Geschwindigkeit von 7 bis 22 m/sec. In allen Beispielen verwendet man eine Strömungsgeschwindigkeit von 9,2 m/sec und homogene Metallprallwände. Der Reinigungseffekt der Prallwände, die Formaldehydausbeute und die Lebensdauer des Katalysators sind jedoch unbefriedigend.

Es wurde nun gefunden, dass man Formaldehyd durch oxidierende Dehydrierung von Methanol im Gemisch mit Wasser in Gegenwart eines Silberkatalysators bei erhöhter Temperatur und nachfolgender Abkühlung und Absorption der heissen Reaktionsgase vorteilhaft erhält, wenn aus

a) Methanol und Wasser mit einer Konzentration von 50 bis 90 Gew.% Methanol, bezogen auf das Gesamtgewicht der beiden Stoffe, in eine Füllkörperkolonne, die Füllkörper mit einer Gesamtschichtdicke von mindestens 50 cm und einer Gesamtober fläche von mindestens 0,5 cm² je cm³ Füllkörperschicht, einen Kreislauf an Flüssigkeit von 15 bis 90 g/min je g/min der Kolonne zugeführtes Methanol, eine Konzentration der Kreislaufflüssigkeit von 10 bis 45 Gew.% Methanol, eine Temperatur am Eingang der Kreislaufflüssigkeit in die Kolonne von 50 bis 86°C und eine von dieser Eingangstemperatur um 10 bis 25°C niedrigere Sumpftemperatur von 40 bis 70°C besitzt, leitet,

b) mit Luft, Inertgas und/oder Abgas einen Anteil an Methanol und Wasser durch Strippen mit einem Durchsatz von 0,5 bis 3 t Methanol und Wasser pro Stunde und m² Kolonnenquerschnitt aus der Füllkörperkolonne abtrennt,

c) das gebildete gasförmige Gemisch von Methanol, Wasser sowie Luft, Inertgas und/oder Abgas mit einer Belastung von 0,5 bis 3 t Methanol pro m² Katalysatorbettquerschnitt und Stunde in Gegenwart eines Silberkatalysators bei einer Temperatur von 550 bis 750°C umsetzt und

d) schliesslich die heissen Reaktionsgase abkühlt und absorbiert, wobei die anfallende Absorptionswärme und gewünschtenfalls Reaktionswärme und/oder Kondensationswärme ganz oder teilweise zur Erhitzung der Kreislaufflüssigkeit verwendet werden.

Weiterhin wurde gefunden, dass man das Verfahren nach der Erfindung vorteilhaft durchführt, wenn Verunreinigungen enthaltendes Methanol verwendet wird und man die Massnahmen der Stufen

a) und b), dann die Massnahmen einer Stufe

e), worin man das gebildete gasförmige Gemisch von Methanol, Wasser sowie Luft, Inertgas und/oder Abgas durch zwei Schichten Drahtfilter mit einem Drahtdurchmesser von 0,1 bis 0,5 mm und einem freien Schichtvolumen von 90 bis 99,5 Vol.%, bezogen auf das Gesamtvolumen einer Drahtschicht, mit einer Strömungsgeschwindigkeit von 7 bis 13 m/sec in der ersten Schicht und von 1 bis 4 m/sec in der zweiten Schicht, leitet, und schliesslich mit dem gasförmigen Gemisch die Massnahmen der Stufen

c) und d) durchführt.

Die Erfindung geht von der Beobachtung aus, dass ein störungsfreier kontinuierlicher Betrieb mit Methanol, insbesondere Rohmethanol, als Ausgangsstoff nicht durch eine Massnahme allein, z.B. besondere Verdampfungs-, Umsetzungs- oder Reinigungsmethoden der Ausgangsstoffe oder Einhaltung einer bestimmten Belastung des Katalysators, erzielt wird, sondern der Kombination der miteinander verbundenen, erfindungsgemässen Merkmale bedarf. Im Vergleich zu den bekannten Verfahren, die reines Methanol verwenden, liefert das Verfahren nach der Erfindung auf einfacherem und wirtschaftlicherem Wege Formaldehyd in guter Ausbeute und Reinheit. Mit Bezug auf die Synthesen mit Rohmethanol als Ausgangsstoff wird Formaldehyd nach dem erfindungsgemässen Verfahren in besserer Ausbeute, Raum-Zeit-Ausbeute und Reinheit hergestellt. Flüssigkeitsnebel, fein verteilte Feststoffe oder Tröpfchen entsprechender Lösungen werden weitgehend abgetrennt. Die vorgenannten Schwierigkeiten und Betriebsstörungen werden vermieden. Entsprechend ist die Lebensdauer des Katalysators verlängert. Eine rasche Vergiftung des Katalysators durch die Reaktionsluft, Rohmethanol bzw. Reaktionswasser und entsprechend eine Anreicherung von Fremdstoffen in der Lösung des Endstoffs werden vermieden. Die erste oder erneute Inbetriebnahme einer Anlage verläuft einfacher, rascher und störungsfreier, die Stillstandszeit der Anlagen, bedingt durch Katalysatorvergiftung oder Störungen des Verdampfers, wird verringert. Da erfindungsgemäss Methanol und Wasser durch Strippen und entsprechend durch Verdunstung und nicht durch Verdampfung aus der Füllkörperkolonne abgetrennt und als gasförmiges Gemisch der Reaktion, gegebenenfalls über die zwei Drahtfilterschichten, zugeführt werden, wird die zur Verdampfung zusätzlich notwendige Energie eingespart. Ebenfalls sind entsprechende Beheizungseinrichtungen der Verdampfung, z.B. Aussenheizung mit zusätzlichem Dampf oder Einleiten von zusätzlichem Dampf in den Verdampfersumpf, nicht notwendig. Andererseits genügt schon die Aufheizung der Kreislaufflüssigkeit durch Wärmeaustausch mit den heissen Reaktionsgasen, Kondensaten und/oder Absorptionslösungen des Formaldehyds, um die erfindungsgemässen Temperaturen für den Strippvorgang (Stripptemperatur) zu erzielen. Somit ist gleichzeitig eine rationelle Verwendung der Reaktionswärme, Absorptionswärme und/oder Kondensationswärme vorteilhaft gegeben.

Alle diese vorteilhaften Ergebnisse sind im Hinblick auf den Stand der Technik überraschend. Im allgemeinen verwendet man gerade im grosstechnischen Betrieb kein analysenreines Methanol, sondern Reinmethanole mit Verunreinigungen unterhalb 0,15 Gew.% und gegebenenfalls Rohmethanole mit vorgenannten höheren Konzentrationen an Verunreinigungen. Im Hinblick auf den Stand der Technik hätte man bei der Strippoperation vermutet, dass erheblich höhere Mengen an Verunreinigung mitgerissen werden und den Katalysator beeinträchtigen, da die Verunreinigungen sich in der Kreislaufflüssigkeit anreichern.

Ebenfalls war es überraschend, dass die Wärmemengen der Reaktion, der Absorption und Kondensation, die die Absorptionslösung erhitzen und bisher durch besondere Kühleinrichtungen in der Absorption entfernt werden mussten, erfindungsgemäss wirtschaftlich in derselben Anlage wiederverwendet werden und für die Bildung des dampfförmigen Methanol-Wasser-Gemischs genügen. Denn die durch Wärmeaustausch mit den im allgemeinen 55 bis 85°C heissen Absorptionslösungen übertragbare Wärmemenge reicht nicht aus, um eine entsprechende Verdampfung des Wasser-Methanol-Gemischs zu erzielen. Es ist daher überraschend, dass auch der Wärmeaustausch mit der Absorptionslösung allein genügt und dazu nur durch Strippen und entsprechende Verdunstung allein die Gas-Dampfphase von Methanol und Wasser gebildet wird, bei dem erfindungsgemässen Verfahren trotzdem vorteilhafte Ergebnisse resultieren und insbesondere auch überschüssige Wärmeenergie (Reaktions- und Kondensationswärme) für andere Verwendungen abgegeben wird. Auch war im Hinblick auf die bekannten Verfahren, die im Sumpf stets eine höhere, dem Kochpunkt von Wasser entsprechende Temperatur und am Kolonnenkopf niedrigere Temperaturen aufweisen, unerwartet, dass die erfindungsgemässen niedrigeren Temperaturen und noch dazu mit einem Temperaturgefälle vom Kolonneneingang (höhere) zum Sumpf (tiefere Temperatur) die vorteilhaften Ergebnisse liefern. Ebenfalls war im Hinblick auf die bekannten Verdampferkolonnen die vorteilhafte Verwendung einer Füllkörperkolonne überraschend.

In Verdampfungskolonnen müssen häufig Anteile der Sumpfflüssigkeit abgetrennt werden, um Anreicherung von Verunreinigungen und damit die vorgenannten Schwierigkeiten der Verdampfung zu verringern. Bei dem erfindungsgemässen Verfahren braucht stündlich auch nur bis höchstens 100 Gew.%, bezogen auf die Gesamtmenge an stündlich abgetrennter Sumpfflüssigkeit bei einem Verdampfungsverfahren, abgetrennt zu werden. Dieses Merkmal des erfindungsgemässen Verfahrens ist gerade auch im Hinblick auf DE-OS 23 23 758 überraschend, denn angesichts der im Vergleich zum Zulauf von Rohmethanol höheren Konzentration der Verunreinigungen in der im oberen Teil der Kolonne eintretenden Kreislaufflüssigkeit musste mit einem höheren Anteil an mitgeschleppten Verunreinigungen in dem Gasgemisch und somit mit schlechteren Ausbeuten und verringerter Lebensdauer des Katalysators gerechnet werden.

Für das Verfahren verwendete Ausgangsstoffe sind Luft, Wasser und nach einem Hochdruck- oder Niederdruckverfahren hergestelltes Methanol. Verwendet man wasserhaltiges Methanol, insbesondere Rohmethanol, kann man die zusätzliche Wasserzugabe ganz oder teilweise einsparen. Zweckmässig führt man anstelle getrennter Zuführung Gemische von Rohmethanol mit Wasser in die Füllkörperkolonne ein; die Konzentration der wässerigen Gemische kann zweckmässig von 50 bis 90, vorzugsweise von 60 bis 85 Gew.% Methanol schwanken; entsprechend werden bei getrennter Zuführung zweckmässig Mengen zwischen 0,11 und 1, vorzugsweise von 0,18 bis 0,67 g Wasser je g Methanol verwendet. Im allgemeinen wird als Verunreinigungen enthaltendes Methanol ein Rohmethanol mit 0,15 bis 6, vorteilhaft 0,15 bis 5, insbesondere 0,15 bis 4 Gew.% Verunreinigungen eingesetzt. Methanol, Wasser und Luft werden als dampf(gas)förmiges Ausgangsgemisch zweckmässig im Verhältnis von 0,25 bis 0,6, vorzugsweise 0,35 bis 0,5 Mol Sauerstoff in Gestalt von Luft je Mol Methanol und 0,1 bis 2, vorzugsweise 0,2 bis 1,2 Mol Sauerstoff in Gestalt von Luft je Mol im Ausgangsgemisch insgesamt enthaltenen Wassers umgesetzt. Die Zuführungsgeschwindigkeit von Flüssigkeit bzw. Luft, Inertgas und/oder Abgas zur Füllkörperkolonne wird entsprechend zweckmässig so geregelt, dass das in den Reaktionsraum eintretende Gemisch eine Zusammensetzung dieser Molverhältnisse aufweist. Man verwendet Durchsätze durch die Füllkörperkolonne von 0,5 bis 3 Tonnen frisch zugeführtes Wasser und Methanol und zweckmässig 1,2 bis 4,5 Tonnen Luft oder 0,4 bis 2,5 Tonnen Inertgas oder 0,4 bis 2,5 Tonnen Abgas oder 1,6 bis 7,0 Tonnen Gemisch von 2 oder allen drei Komponenten Luft, Inertgas, Abgas pro Stunde und Quadratmeter Kolonnenquerschnitt.

Man kann zum Strippen in der Füllkörperkolonne drei Komponenten (Strippgas), nämlich Luft, Inertgas und Abgas, sowohl jede Komponente allein oder ein Gemisch von 2 oder allen drei Komponenten als Strippgas verwenden. Bevorzugt sind b1) Luft oder b2) ein Gemisch von Luft und Abgas als Strippgas. Man kann bei Gemischen als Strippgas jede vorgenannte Komponente getrennt von der anderen oder in Form des Gemischs in die Füllkörperkolonne einführen. Man kann auch von den drei Komponenten, zweckmässig von Luft, einen Anteil zum Strippen und den restlichen Anteil erst nach dem Durchgang durch die Kolonne und gegebenenfalls Drahtschichten, zweckmässig vor dem Reaktoreintritt in das gas(dampf)förmige Ausgangsgemisch eingeben. In einer bevorzugten Ausführungsform führt man ein Gemisch von Luft und Abgas als Strippgas durch die Kolonne und gibt einen weiteren Anteil an Luft, zweckmässig 3 bis 20 Gew.%, bezogen auf die Gesamtmenge an verwendeter Luft, zum gas(dampf)förmigen Ausgangsgemisch (Luft, Abgas, Methanol und Wasser). Man wählt zweckmässig 20 bis 100, vorteilhaft 40 bis 80 Gew.% Inertgas, bevorzugt Stickstoff, und/oder 20 bis 100, vorteilhaft 40 bis 80 Gew.% Abgas, bezogen auf die gesamte Gewichtsmenge Luft. In einer zweckmässigen Ausführungsform lässt man einen Teil des Abgases der erfindungsgemässen Oxidation entweichen und führt den anderen in den Reaktionskreislauf zurück. Der Anteil des Abgases, der zweckmässig mit Luft vermischt und so der Reaktion wieder zugeführt wird, beträgt zweckmässig 20 bis 110, insbesondere 44 bis 88 Gew.%, bezogen auf den Anteil an die Anlage verlassende Abgas. Das Abgas enthält im wesentlichen Stickstoff, Wasserstoff, Kohlendioxid, Kohlenmonoxid, Wasser, Methanol, Argon und in der Regel von 0,1 bis 0,5 g Formaldehyd im Kubikmeter Abgas. Bei grösseren Umsetzungsmengen kann man auch als Abgas das einer anderen Formaldehydherstellung durch oxidierende Dehydrierung des Methanols, vorzugsweise in Gegenwart von Silberkatalysatoren oder von Metalloxiden, z.B. von Oxiden des Eisens und des Molybdäns, entnommene Abgas verwenden.

Die Füllkörperkolonne hat zweckmässig eine Länge von 2 bis 15, insbesondere von 3 bis 10 m, einen Durchmesser von 0,5 bis 5, insbesondere 1 bis 3,5 m und besitzt Füllkörper, vorteilhaft mit einem Durchmesser des einzelnen Füllkörpers von 15 bis 150, insbesondere 25 bis 80 mm. Die Gesamtschichtdicke (Füllhöhe) der Füllkörperschicht beträgt mindestens 50, zweckmässig 100 bis 1000, insbesondere 300 bis 800 cm. Die Gesamtoberfläche der Füllkörper beträgt mindestens 0,5, zweckmässig 0,5 bis 2,2, vorzugsweise 0,9 bis 1,6 $cm^2$ je Kubikzentimeter Füllkörperschicht. Als Füllkörper können z.B. Raschigringe, Intosringe, Prymringe, Pallringe, Berlsättel, Intaloxsättel, Torussättel, Interpackkörper, Stedmankörper, Schrägfilmblätter, Drahtnetzringe, Haltmeier-Rollen, Zwillingskörper, Wilsonspiralen oder Braunschweiger Wendeln verwendet werden.

In der Regel werden Methanol und Wasser, getrennt oder im allgemeinen als Gemisch, vom Kolonnenfuss her, Luft, Inertgas und/oder Abgas, gegebenenfalls im Gemisch, vom Kolonnenfuss her eingeführt; in einer bevorzugten Ausführungsform werden Luft bzw. vorgenannte Luftgemische durch den Kolonnensumpf geleitet. Die Kolonnen besitzen einen Flüssigkeitskreislauf, der in der Regel vom Kolonnensumpf ausgeht und am Kopf der Kolonne, zweckmässig von 30 bis 200 cm unterhalb des Kolonnenkopfes endet. Die Kreislaufflüssigkeit hat zweckmässig einen Durchsatz von 3000 bis 6000, insbesondere von 3500 bis 5500 g je $cm^2$ Kolonnenquerschnitt und Stunde, und wird in der Regel über Wärmeaustauscher geführt, worin sie als Kühlflüssigkeit für die heissen Reaktionsgase, Kondensate und Absorptionslösungen dient. Gegebenenfalls kann ein Teil des Wärmeaustauschs der Kreislaufflüssigkeit mit den heissen Reaktionsgasen und/oder Absorptionslösungen einer anderen Formaldehydsynthese bzw. einer Formaldehydfolgereaktion oder den Wärmeüberträgern einer anderen Synthese erfolgen. In allen Fällen muss zumindest ein Teil, zweckmässig 40 bis 70, bevorzugt 50 bis 60% (bezogen auf die gesamte, dem Flüssigkeitskreislauf zugeführte Wärme) der Absorptionswärme, die man bei der Absorption von Formaldehyd aus den heissen Reaktionsgasen und/oder Kondensaten, zweckmässig in Wasser oder wässeriger Harnstofflösung, erhält, zur Erhitzung des Flüssigkeitskreislaufs dienen. Die wässerige Harnstofflösung enthält zweckmässig 50 bis 70, bevorzugt 65 bis 70 Gew.% Harnstoff.

Zweckmässig stellt die Kreislaufflüssigkeit ein wässeriges Methanolgemisch dar, das 10 bis 45, insbesondere 15 bis 40 Gew.% Methanol (berechnet als Methanol 100%) und 0,1 bis 15, insbesondere 1 bis 10 Gew.% Verunreinigungen enthält. Der Kreislauf führt der Kolonne Flüssigkeit in einem Verhältnis von 15 bis 90, insbesondere 20 bis 70 g (je Minute) je Gramm pro Minute der Kolonne zugeführtes frisches Methanol zu und besitzt eine Temperatur am Eingang in die Kolonne von 50 bis 86, insbesondere 60 bis 80°C und eine Temperatur im Kolonnensumpf von 40 bis 70, insbesondere 45 bis 60°C. Die Sumpftemperatur ist 10 bis 25, zweckmässig 15 bis 20°C niedriger als die Eingangstemperatur der Kreislaufflüssigkeit. Gegebenenfalls kann man einen kleinen Teil der Kreislaufflüssigkeit, zweckmässig von 0,01 bis 0,1 Gew.% des Kreislaufflüssigkeitsstroms zur Entfernung hoher Mengen von Verunreinigungen abtrennen und verwerfen, wobei gewünschtenfalls das in diesem Teil enthaltene Methanol und Wasser über einen Verdampfer oder Stripper isoliert und der Reaktion wieder zugeführt werden. Zur Verdampfung dieser Anteile kann zweckmässig ebenfalls die bei der Abkühlung der heissen Reaktionsgase freiwerdende Reaktions- bzw. Kondensationswärme verwendet werden, z.B. in Gestalt von durch Wärmeaustausch erzeugtem Überschussdampf.

Luft, Inertgas und/oder Abgas dienen als Schleppmittel (Strippmittel) für das der Kolonne zugeführte Gemisch an Methanol und Wasser, für die Kreislaufflüssigkeit und, sofern die Gase durch den Sumpf geleitet werden, für die Sumpfflüssigkeit; die Sumpfflüssigkeit entspricht in der Zusammensetzung der Komponenten der Kreislaufflüssigkeit am Eingang in die Kolonne. Die geschilderte Verfahrensweise wird hier, entsprechend einer Definition in «Introduction to Chemical Enginee-

ring» von W.L. Badger und J.T. Banchero (McGraw-Hill Book Comp. Inc. 1955), Seite 437 (letzter Absatz), als Strippen bezeichnet. Die Gaszuführung wird so gewählt, dass sich ein Gleichgewicht zwischen vorgenannten Durchsätzen an Wasser/Methanol-Gemisch, Kreislaufflüssigkeit und Gas und den vorgenannten Mengenverhältnissen der Komponenten Luft, Inertgas, Abgas, Methanol und Wasser bei der Umsetzung einstellt. Der in der Kolonne durch Strippen abgetrennte Anteil an Methanol und Wasser ist in der Hauptsache gasförmig und besitzt im allgemeinen weniger als 0,1 Gew.% flüssiges Methanol-Wasser-Gemisch und im allgemeinen weniger als 0,01 Gew.% flüssige und feste Verunreinigungen.

In der Gasphase stellt sich im allgemeinen ein Verhältnis von 50 bis 90, insbesondere 60 bis 85 Gew.% Methanol (berechnet 100%), bezogen auf die Gesamtmenge an Wasser und Methanol in dieser Phase ein. Das gasförmige Gemisch wird im Falle der Verwendung von Rohmethanol nach den Stufen a) und b) zweckmässig in der Stufe e) nun durch 2 Schichten Drahtfilter geleitet. Unter Drahtfilter werden hier Drahtgebilde verstanden, die in beliebiger Weise, regelmässig oder unregelmässig, z.B. als Gewebe, Gewirke, in Form von Netzen, Wendeln, Spiralen strukturiert sein können und zu zwei voneinander getrennten Schichten, z.B. in zwei Packungen, vereinigt werden. Der Draht in beiden Schichten hat einen Durchmesser (Drahtstärke) von 0,1 bis 0,5, vorteilhaft 0,1 bis 0,3 mm, beide Schichten haben jeweils ein freies Schichtvolumen, das hier als das nicht vom Drahtvolumen besetzte Gesamtvolumen der Schicht definiert wird, von 90 bis 99,5 insbesondere 95 bis 99,1 Vol.%, bezogen auf das Gesamtvolumen der Schicht. Der Gasstrom durchströmt die erste Schicht mit einer Geschwindigkeit von 7 bis 13, insbesondere 9 bis 11 m/sec. und zweckmässig mit einer Belastung von 12 bis 60, insbesondere 20 bis 51 g Methanol pro Minute und cm² Schichtquerschnitt und die 2. Schicht mit einer Geschwindigkeit von 1 bis 4, insbesondere 1,5 bis 3,5 m/sec. und zweckmässig mit einer Belastung von 1 bis 20, insbesondere 1,5 bis 18 g Methanol je Minute und cm² Schichtquerschnitt. Die Schichtdicke der zwei Schichten beträgt zweckmässig jeweils 10 bis 40, insbesondere 20 bis 30 cm je Schicht.

Im Falle von Reinmethanol zweckmässig nach Stufe b) ohne Stufe e), oder im Falle von Rohmethanol bevorzugt nach Durchgang durch die zwei Drahtschichten wird in c) das gasförmige Gemisch dem Reaktor mit dem Silberkatalysator zugeführt. Das Gemisch enthält jetzt nur noch Verunreinigungen in einer Menge unter 2, im allgemeinen von 0,01 bis 1 Gew.%, bezogen auf die Gesamtverunreinigung im Rohmethanol und 0,00014 bis 0,014 Gew.%, bezogen auf das gasförmige Methanol (berechnet 100%).

Für das Verfahren nach der Erfindung kommen die für die Herstellung von Formaldehyd im allgemeinen verwendeten Silberkatalysatoren in Betracht, z.B. die in DE-AS 12 31 229, DE-PS 23 22 757, Ullmanns Encyclopädie der technischen Chemie (3. Auflage), Band 7, Seiten 656 ff. und in den zum Stand der Technik genannten Veröffentlichungen beschriebenen. Vorzugsweise verwendet man Zwei- und Mehrschicht-Silberkatalysatoren. Bezüglich Herstellung des Katalysators und Durchführung der entsprechenden Umsetzung mit diesen Katalysatoren wird auf die genannten Veröffentlichungen verwiesen. In einer bevorzugten Ausführungsform wird die Umsetzung mit einem Katalysator mit der Gesamtschichtdicke von 15 bis 35 mm und 3 oder mehr Schichten Silberkristallen durchgeführt, wobei ein Teil der Schichten 72,5 bis 89 Gew.% des Katalysators mit Teilchen der Korngrösse 1 bis 2,5 mm, ein Teil der Schichten 2,5 bis 7,5 Gew.% des Katalysators mit Teilchen der Korngrösse 0,75 bis 1 mm und der restliche Teil der Schichten 8,5 bis 20 Gew.% des Katalysators mit Teilchen der Korngrösse 0,2 bis 0,75 mm enthalten. Bevorzugte Katalysatoren sind die in der deutschen Patentschrift 23 22 757 beschriebenen.

Die Belastung während der Oxidation beträgt 0,5 bis 3, insbesondere 1 bis 2,50 t Methanol (berechnet 100%) je m² Katalysatorbettquerschnitt und Stunde.

Die Oxidation wird im übrigen in bekannter Weise durchgeführt, indem man z.B. das Gasgemisch aus Methanoldampf, Luft, Wasserdampf und gegebenenfalls Inertgas und/oder Abgas in vorgenannten Mengen bei Temperaturen von 550 bis 750° C, insbesondere 600 bis 700° C, durch den Silberkatalysator leitet. Die Umsetzung wird im allgemeinen bei Drücken zwischen 0,5 und 2 bar, vorzugsweise zwischen 0,8 und 1,8 bar, kontinuierlich durchgeführt. Es ist dann in Stufe d) zweckmässig, die die Katalysatorzone verlassenden Reaktionsgase innerhalb kurzer Zeit, beispielsweise in weniger als 1/10 Sekunden, abzukühlen, z.B. auf Temperaturen von 150 bis 350° C. Das abgekühlte Gasgemisch wird dann zweckmässig d1) einem Kondensator zur weiteren Abkühlung und dann der Absorption oder d2) direkt ohne Kondensation einem Absorptionsturm zugeführt, in welchem der Formaldehyd in der Regel mit Wasser oder wässerigen Harnstofflösungen, vorteilhaft im Gegenstrom, aus dem Gasgemisch gewaschen wird.

Zwischen Reaktor und Absorptionsturm liegt zweckmässig der vorgenannte Wärmeaustauscher von Reaktionsgas und/oder Kondensat und Kreislaufflüssigkeit, im Kreislauf der Absorptionsanlage der vorgenannte Wärmeaustauscher von Kreislaufflüssigkeit und heisser Absorptionslösung. Vorteilhaft verwendet man zur Temperatureinstellung der Kreislaufflüssigkeit eine Wärmemenge, die zu 10 bis 100, insbesondere 40 bis 100% aus dem Wärmeaustausch mit der Absorption, 0 bis 90, insbesondere 0 bis 60% aus der Kondensation und 0 bis 50, insbesondere 0 bis 30% aus der Abkühlung der Reaktionsgase stammt.

Der nach dem Verfahren der Erfindung herstellbare Formaldehyd ist Desinfektionsmittel, Gerbstoff, Reduktionsmittel und wertvoller Ausgangsstoff für die Herstellung von Kunstharzen, Klebmitteln und Kunststoffen. Bezüglich der Verwendung wird auf den genannten Band 12 von Ullmann (3. Auflage), Seite 670, verwiesen.

*Beispiel 1:*

(Zeichnung 1)

22 030 kg Luft wurden stündlich durch Leitung (1) der Füllkörperkolonne (2) unterhalb der Oberfläche des Kolonnensumpfes zugeführt. Im Gegenstrom wurden stündlich 17 310 kg eines Rohmethanol-Wasser-Gemisches (1 Gew.% Verunreinigung, 69,6 Gew.% Methanol, 29,4 Gew.% Wasser) über Leitung (3) sowie 310 000 kg Sumpf (Kreislaufflüssigkeit) der Füllkörperkolonne über Leitung (4), Pumpe (5), Leitung (6), Wärmeaustauscher (7), Leitung (8), Kondensator (9) und Leitung (10) geführt. Die Füllkörperkolonne war 12,8 m lang, hatte einen Durchmesser von 300 cm und enthielt 42 m³ Pallringe als Füllkörper (3,5 cm Länge, 3,5 cm Durchmesser). Der Sumpf (Kreislaufflüssigkeit) enthält 10 Gew.% Verunreinigung, 28 Gew.% Methanol und 62 Gew.% Wasser. Im Wärmeaustauscher (7) wurde die Kreislaufflüssigkeit im Gegenstrom indirekt mit Absorptionsflüssigkeit über Leitung 19 auf eine Temperatur von 68°C erwärmt. Im Kondensator (9) wurde die Kreislaufflüssigkeit indirekt im Wärmeaustausch mit heissen Reaktionsgasen (über Leitung (20)) auf 79°C erhitzt. Die Temperatur am Eingang der Kreislaufflüssigkeit in die Kolonne (2) betrug 78°C, die Sumpftemperatur der Kolonne (2) 62°C. Über Leitung (13) wurden stündlich 1730 kg Kreislaufflüssigkeit abgezogen und im Verdampfer (14) zusammen mit stündlich 3343 kg Wasser verdampft. Über den Sumpfabzug (15) wurden stündlich 572 kg Verdampfersumpf (172 kg Verunreinigungen, 400 kg Wasser) entzogen. Der stündlich verdampfte Anteil (485 kg Methanol, 4015,99 kg Wasser, 0,01 kg Verunreinigung) wurde aus Verdampfer (14) über Leitung (25) der Leitung (11) zugeführt. Der Durchsatz durch die Füllkörperkolonne betrug 0,24 kg Rohmethanol-Wassergemisch und 0,31 kg Luft pro Stunde und cm² Kolonnenquerschnitt. Die zugeführte Luft wurde durch die Füllkörperkolonne (2), wobei sie einen Anteil an Methanol und Wasser strippte, und dann das resultierende Dampf-Luft-Gemisch durch zwei Schichten Drahtfilter (29) geführt. Als Schicht wurde jeweils eine Packung Drahtgewirke aus nichtrostendem Chrom-Nickelstahl verwendet. In jeder Schicht betrug der Drahtdurchmesser 0,15 mm, das freie Schichtvolumen 99 Vol.%, bezogen auf das Gesamtvolumen der Schicht. Die Strömungsgeschwindigkeit des Gas-Dampf-Gemischs betrug in der ersten Schicht 12,8 m/sec, in der zweiten Schicht 1,2 m/sec. Bei dem Durchgang des Gas-Dampf-Gemisches durch die zwei Schichten (29) wurde stündlich ein Anteil (6 kg) abgeschieden und in die Füllkörperkolonne (2) über Leitung (16) zurückgeführt.

· Man verwendete eine Anlage mit einem senkrechten Rohrreaktor (12), der an seinem Kopf die Zuführung des gasförmigen Ausgangsgemischs und die Reaktorhaube enthielt. Die Katalysatorschicht (27) lag unterhalb des Reaktorkopfes.

Weiter unten folgte eine Kühlzone, die mit den Leitungen (17) und (18) verbunden war.

In den Reaktor wurde ein Katalysator aus Silberkristallen (429 kg) folgender Zusammensetzung eingetragen:

| | Anteil am Katalysator (Gew.%) | Korngrösse mm |
|---|---|---|
| Schicht 1 | 12,9 | 0,4 -0,75 |
| Schicht 2 | 1,2 | 0,2 -0,4 |
| Schicht 3 | 5,3 | 0,75-1 |
| Schicht 4 | 14,1 | 1 -1,75 |
| Schicht 5 | 66,5 | 1 -2,5 |

Schicht 2 wurde als Ringschicht in der Randzone des Katalysators auf Schicht 3 aufgestreut. Der Durchmesser des Katalysators war 310 cm, die lichte Weite der Ringschicht 305 cm. Dem Reaktor wurden aus der 2. Drahtschicht (29) über Leitung 11 stündlich 42 111 kg gasförmiges Gemisch, das 28,6 Gew.% Methanol, 0,0026 Gew.% Verunreinigungen, 19,0974 Gew.% Wasser und 52,3 Gew.% Luft enthielt, zugeführt. Das Gemisch wurde durch den Katalysator geleitet und bei 700°C und 1,4 bar umgesetzt.

Zur Abkühlung der Reaktionsgase im Reaktor (12) wurden über Leitung 17 stündlich 14 600 kg Kesselspeisewasser zugeführt und über Leitung 18 stündlich 14 600 kg Wasserdampf (16 bar) als Überschuss, der für eine andere Umsetzung verwendet wurde, abgegeben. Die auf 152°C abgekühlten Reaktionsgase wurden über Leitung 20 dem Kondensator (9) und dann über die Leitung (21) dem Absorptionsturm (22) zugeführt. Sie wurden in ihm absorbiert, wobei über Leitung (23) stündlich 2800 kg Wasser zugeführt wurden. Der Turm (22) besass einen Kreislauf der Absorptionsflüssigkeit über Leitung (19), Austauscher (7) und Leitung (26). Durch die heissen Gase im Kondensator (9), die selbst auf 79°C abgekühlt wurden, und die 70°C warme Absorptionslösungen im Austauscher (7) wurde die Kreislaufflüssigkeit der Füllkörperkolonne erwärmt. Über Leitung (28) wurde das Abgas abgeführt. Über Leitung (24) wurden stündlich in Form einer 40 gew.%igen wässerigen Formaldehydlösung 10 000 kg Formaldehyd (ber. 100%), entsprechend einer Ausbeute von 88,5% der Theorie abgetrennt. Die Lebensdauer des Katalysators betrug 116 Tage. Die Formaldehydlösung hatte einen Gehalt von 3,8 Gew.% Methanol und 0,025 Gew.% Ameisensäure, bezogen auf Formaldehyd (ber. 100%).

*Beispiel 2:*

(Zeichnung 1)

22 030 kg Luft wurden stündlich durch Leitung (1) der Füllkörperkolonne (2) unterhalb der Oberfläche des Kolonnensumpfes zugeführt. Im Gegenstrom wurden stündlich 17 310 kg eines Rohmethanol-Wasser-Gemisches (1 Gew.% Verunreinigung, 69,6 Gew.% Methanol, 29,4 Gew.%

Wasser) über Leitung (3) sowie 310 000 kg Sumpf (Kreislaufflüssigkeit) der Füllkörperkolonne über Leitung (4), Pumpe (5), Leitung (6), Wärmeaustauscher (7), Leitung (8), Kondensator (9) und Leitung (10) geführt. Die Füllkörperkolonne war 12,8 m lang, hatte einen Durchmesser von 300 cm und enthielt 42 m³ Pallringe als Füllkörper (3,5 cm Länge, 3,5 cm Durchmesser). Der Sumpf (Kreislaufflüssigkeit) enthält 10 Gew.% Verunreinigung, 28 Gew.% Methanol und 62 Gew.% Wasser. Im Wärmeaustauscher (7) wurde die Kreislaufflüssigkeit im Gegenstrom indirekt mit Absorptionsflüssigkeit über Leitung 19 auf eine Temperatur von 68° C erwärmt. Im Kondensator (9) wurde die Kreislaufflüssigkeit indirekt im Wärmeaustausch mit heissen Reaktionsgasen (über Leitung (20)) auf 79° C erhitzt. Die Temperatur am Eingang der Kreislaufflüssigkeit in die Kolonne (2) betrug 78° C, die Sumpftemperatur der Kolonne (2) 62° C. Über Leitung (13) wurden stündlich 1730 kg Kreislaufflüssigkeit abgezogen und im Verdampfer (14) zusammen mit stündlich 3343 kg Wasser verdampft. Über den Sumpfabzug (15) wurden stündlich 572 kg Verdampfersumpf (172 kg Verunreinigungen, 400 kg Wasser) entzogen. Der stündlich verdampfte Anteil (485 kg Methanol, 4015,99 kg Wasser, 0,01 kg Verunreinigung) wurde aus Verdampfer (14) über Leitung (25) der Leitung (11) zugeführt. Der Durchsatz durch die Füllkörperkolonne betrug 0,24 kg Rohmethanolwassergemisch und 0,31 kg Luft pro Stunde und cm² Kolonnenquerschnitt. Die zugeführte Luft wurde durch die Füllkörperkolonne (2), wobei sie einen Anteil an Methanol und Wasser strippte, und dann das resultierende Dampf-Luft-Gemisch durch zwei Schichten Drahtfilter (29) geführt. Als Schicht wurde jeweils eine Packung Drahtgewirke aus nichtrostendem Chrom-Nickelstahl verwendet. In jeder Schicht betrug der Drahtdurchmesser 0,28 mm, das freie Schichtvolumen 99 Vol.%, bezogen auf das Gesamtvolumen der Schicht. Die Strömungsgeschwindigkeit des Gas-Dampf-Gemischs betrug in der ersten Schicht 7 m/sec, in der zweiten Schicht 3,9 m/sec. Bei dem Durchgang des Gas-Dampf-Gemisches durch die zwei Schichten wurde stündlich ein Anteil (6 kg) abgeschieden und in die Füllkörperkolonne (2) über Leitung (16) zurückgeführt.

Man verwendete eine Anlage mit einem senkrechten Rohrreaktor (12), der an seinem Kopf die Zuführung des dampfförmigen Ausgangsgemischs und die Reaktorhaube enthielt. Die Katalysatorschicht (27) lag unterhalb des Reaktorkopfes. Weiter unten folgte eine Kühlzone, die mit den Leitungen (17) und (18) verbunden war.

In den Reaktor wurde ein Katalysator aus Silberkristallen (429 kg) folgender Zusammensetzung eingetragen:

| | Anteil am Katalysator (Gew.%) | Korngrösse mm |
|---|---|---|
| Schicht 1 | 12,9 | 0,4 -0,75 |
| Schicht 2 | 1,2 | 0,2 -0,4 |

| | Anteil am Katalysator (Gew.%) | Korngrösse mm |
|---|---|---|
| Schicht 3 | 5,3 | 0,75-1 |
| Schicht 4 | 14,1 | 1 -1,75 |
| Schicht 5 | 66,5 | 1 -2,5 |

Schicht 2 wurde als Ringschicht in der Randzone des Katalysators auf Schicht 3 aufgestreut. Der Durchmesser des Katalysators war 310 cm, die lichte Weite der Ringschicht 305 cm. Dem Reaktor wurden aus der 2. Drahtschicht (29) über Leitung 11 stündlich 42 111 kg gasförmiges Gemisch, das 28,6 Gew.% Methanol, 0,0026 Gew.% Verunreinigungen, 19,0974 Gew.% Wasser und 52,3 Gew.% Luft enthielt, zugeführt. Das Gemisch wurde durch den Katalysator geleitet und bei 700° C und 1,4 bar umgesetzt.

Zur Abkühlung der Reaktionsgase im Reaktor (12) wurden über Leitung (17) stündlich 14 600 kg Kesselspeisewasser zugeführt und über Leitung (18) stündlich 14 600 kg Wasserdampf (16 bar) als Überschuss, der für eine andere Umsetzung verwendet wurde, abgegeben. Die auf 152° C abgekühlten Reaktionsgase wurden über Leitung 20 dem Kondensator (9) und dann über die Leitung (21) dem Absorptionsturm (22) zugeführt. Sie wurden in ihm absorbiert, wobei über Leitung (23) stündlich 2800 kg Wasser zugeführt wurden. Der Turm (22) besass einen Kreislauf der Absorptionsflüssigkeit über Leitung (19), Austauscher (7) und Leitung (26). Durch die heissen Gase im Kondensator (9), die selbst auf 79° C abgekühlt wurden, und die 70° C warme Absorptionslösungen im Austauscher (7) wurde die Kreislaufflüssigkeit der Füllkörperkolonne erwärmt. Über Leitung (28) wurde das Abgas abgeführt. Über Leitung 24 wurden stündlich in Form einer 40 gew.%igen wässerigen Formaldehydlösung 10 000 kg Formaldehyd (ber. 100%), entsprechend einer Ausbeute von 88,5% der Theorie abgetrennt. Die Lebensdauer des Katalysators betrug 116 Tage. Die Formaldehydlösung hatte einen Gehalt von 3,2 Gew.% Methanol und 0,022 Gew.% Ameisensäure, bezogen auf Formaldehyd (ber. 100%).

*Beispiel 3:*

(Zeichnung 2)

5885 kg Luft wurden stündlich durch Leitung (1) und 3290 kg Abgas (1,3 Gew.% $H_2$, 0,5 Gew.% CO, 6,9 Gew.% $CO_2$) wurden stündlich durch Leitung (30) und Pumpe (31) der Füllkörperkolonne (2) unterhalb der Oberfläche des Kolonnensumpfes zugeführt. Im Gegenstrom wurden stündlich 4050 kg eines Rohmethanol-Wasser-Gemisches (0,3 Gew.% Verunreinigung, 75,8 Gew.% Methanol, 23,9 Gew.% Wasser) über Leitung (3) sowie 117 000 kg Sumpf (Kreislaufflüssigkeit) der Füllkörperkolonne über Leitung (4), Pumpe (5), Leitung (6), Wärmeaustauscher (7) und Leitung (8) geführt. Die Füllkörperkolonne war 9 m lang, hatte einen Durchmesser von 180 cm und enthielt 12,5 m³ Pallringe als Füllkörper (3,5 cm Länge, 3,5 cm Durchmesser). Der Sumpf (Kreislaufflüssigkeit) enthielt 10 Gew.% Verunreinigung, 33,3 Gew.% Methanol und 56,7 Gew.% Wasser. Im Wärmeaustauscher (7) wurde die Kreislaufflüssigkeit im Gegenstrom indirekt mit Absorptionsflüssigkeit über Leitung 19 auf eine Temperatur von 59°C erwärmt. Die Temperatur am Eingang der Kreislaufflüssigkeit in die Kolonne (2) betrug 59°C, die Sumpftemperatur der Kolonne (2) 49°C. Über Leitung (13) wurden stündlich 120 kg Kreislaufflüssigkeit abgezogen (40 kg Methanol, 68 kg Wasser, 12 kg Verunreinigung). Der Durchsatz durch die Füllkörperkolonne betrug 0,16 kg frisch zugeführtes Rohmethanol-Wassergemisch, 0,23 kg Luft und 0,13 kg Abgas pro Stunde und cm² Kolonnenquerschnitt. Die zugeführte Luft und der zugeführte Anteil Abgas wurden duch die Füllkörperkolonne (2), wobei sie einen Anteil an Methanol und Wasser strippen, und dann das resultierende Dampf-Gas-Gemisch durch zwei Schichten Drahtfilter (29) geführt. Als Schicht wurde jeweils eine Packung Drahtgewirke aus nichtrostendem Chrom-Nickelstahl verwendet. In jeder Schicht betrug der Drahtdurchmesser 0,4 mm, das freie Schichtvolumen 95 Vol.%, bezogen auf das Gesamtvolumen der Schicht. Die Strömungsgeschwindigkeit des Gas-Dampf-Gemisches betrug in der ersten Schicht 13 m/sec, in der zweiten Schicht 4 m/sec. Bei dem Durchgang des Gas-Dampf-Gemisches durch die zwei Schichten (29) wurde stündlich ein Anteil (2 kg) abgeschieden und in die Füllkörperkolonne (2) über Leitung (16) zurückgeführt.

Man verwendete eine Anlage mit einem senkrechten Rohrreaktor (12), der an seinem Kopf die Zuführung des dampfförmigen Ausgangsgemischs und die Reaktorhaube enthielt. Die Katalysatorschicht (27) lag unterhalb des Reaktorkopfes. Weiter unten folgte eine Kühlzone, die mit den Leitungen (17) und (18) verbunden war.

In den Reaktor wurde ein Katalysator aus Silberkristallen (187 kg) folgender Zusammensetzung eingetragen:

| | Anteil am Katalysator (Gew.%) | Korngrösse mm |
|---|---|---|
| Schicht 1 | 12,9 | 0,4 -0,75 |
| Schicht 2 | 1,2 | 0,2 -0,4 |
| Schicht 3 | 5,3 | 0,75-1 |
| Schicht 4 | 14,1 | 1 -1,75 |
| Schicht 5 | 66,5 | 1 -2,5 |

Schicht 2 wurde als Ringschicht in der Randzone des Katalysators auf Schicht 3 augestreut. Der Durchmesser des Katalysators war 170 cm, die lichte Weite der Ringschicht 160 cm. Dem Reaktor wurden aus der 2. Drahtschicht (29) über Leitung (11) stündlich 13 104 kg gasförmiges Gemisch, das neben Abgas 23 Gew.% Methanol, 0,0014 Gew.% Verunreinigungen, 8,8 Gew.% Wasser und 44,9 Gew.% Luft enthielt, zugeführt.

Das Gemisch wurde durch den Katalysator geleitet und bei 700°C und 1,3 bar umgesetzt.

Zur Abkühlung der Reaktionsgase im Reaktor (12) wurden über Leitung (17) stündlich 4710 kg Kesselspeisewasser zugeführt und über Leitung (18) stündlich 4710 kg Wasserdampf (5 bar) als Überschuss, der für eine andere Umsetzung verwendet wurde, abgegeben. Die auf 180°C abgekühlten Reaktionsgase wurden über Leitung (20) dem Absorptionsturm (22) zugeführt. Sie wurden in ihm absorbiert, wobei über Leitung (23) stündlich 199 kg Wasser zugeführt wurden. Der Turm (22) besass einen Kreislauf der Absorptionsflüssigkeit über Leitung (19), Austauscher (7) und Leitung (26). Durch die 76°C warme Absorptionslösungen in Leitung (19) wurde die Kreislaufflüssigkeit der Füllkörperkolonne erwärmt. Über Leitung (28) wurde das Abgas abgeführt. Über Leitung (24) wurden stündlich in Form einer 50 gew.%igen wässerigen Formaldehydlösung 2546 kg Formaldehyd (ber. 100%), entsprechend einer Ausbeute von 88,5% der Theorie abgetrennt. Die Lebensdauer des Katalysators betrug 116 Tage. Die Formaldehydlösung hatte einen Gehalt von 2,4 Gew.% Methanol und 0,02 Gew.% Ameisensäure, bezogen auf Formaldehyd (ber. 100%).

*Beispiel 4:*

(Zeichnung 2)

5885 kg Luft wurden stündlich durch Leitung (1) und 3290 kg Abgas (Zusammensetzung analog Beispiel 3) wurden stündlich durch Leitung (30) und Pumpe (31) der Füllkörperkolonne (2) unterhalb der Oberfläche des Kolonnensumpfes zugeführt. Im Gegenstrom wurden stündlich 4050 kg eines Rohmethanol-Wasser-Gemisches (0,3 Gew.% Verunreinigung, 75,8 Gew.% Methanol, 23,9 Gew.% Wasser) über Leitung (3) sowie 117 000 kg Sumpf (Kreislaufflüssigkeit) der Füllkörperkolonne über Leitung (4), Pumpe (5), Leitung (6), Wärmeaustauscher (7) und Leitung (8) geführt. Die Füllkörperkolonne war 9 m lang, hatte einen Durchmesser von 180 cm und enthielt im unteren Bereich 2500 l Pallringe als Füllkörper (5 cm, Länge, 5 cm Durchmesser) und darüber 10 000 l Pallringe als Füllkörper (3,5 cm Länge, 3,5 cm Durchmesser). Der Sumpf (Kreislaufflüssigkeit) enthielt 10 Gew.% Verunreinigung, 33,3 Gew.% Methanol und 56,7 Gew.% Wasser. Im Wärmeaustauscher (7) wurde die Kreislaufflüssigkeit im Gegenstrom indirekt mit Absorptionsflüssigkeit über Leitung 19 auf eine Temperatur von 59°C erwärmt. Die Temperatur am Eingang der Kreislaufflüssigkeit in die Kolonne (2) betrug 59°C, die Sumpftemperatur der Kolonne (2) 49°C. Über Leitung (13) wurden stündlich 120 kg Kreislaufflüssigkeit abgezogen (12 kg Verunreinigungen, 68 kg Wasser, 40 kg Methanol). Der Durchsatz durch die Füllkörperkolonne betrug 0,16 kg frisch zugeführtes Rohmethanol Wassergemisch, 0,23 kg Luft und 0,13 kg Abgas pro Stunde und cm² Kolonnenquerschnitt. Die zugeführte Luft wurde durch die Füllkörperkolonne (2), wobei sie einen Anteil an Methanol und Wasser strippte, und dann das resultierende Dampf-Luft-Gemisch durch zwei Schichten Drahtfilter (30) geführt. Als Schicht wurde jeweils eine Packung Drahtgewirke aus nichtrostendem Chrom-Nickelstahl verwendet. In jeder Schicht betrug der Drahtdurchmesser 0,3 mm, das freie Schichtvolumen 95 Vol.%, bezogen auf das Gesamtvolumen der Schicht. Die Strömungsgeschwindigkeit des Gas-Dampf-Gemischs betrug in der ersten Schicht 7 m/sec, in der zweiten Schicht 1 m/sec. Bei dem Durchgang des Gas-Dampf-Gemisches durch die zwei Schichten (29) wurde stündlich ein Anteil (2 kg) abgeschieden und in die Füllkörperkolonne (2) über Leitung (16) zurückgeführt.

Man verwendete eine Anlage mit einem senkrechten Rohrreaktor (12), der an seinem Kopf die Zuführung des dampfförmigen Ausgangsgemischs und die Reaktorhaube enthielt. Die Katalysatorschicht (27) lag unterhalb des Reaktorkopfes. Weiter unten folgte eine Kühlzone, die mit den Leitungen (17) und (18) verbunden war.

In den Reaktor wurde ein Katalysator aus Silberkristallen (187 kg) folgender Zusammensetzung eingetragen:

| | Anteil am Katalysator (Gew.%) | Korngrösse mm |
|---|---|---|
| Schicht 1 | 12,9 | 0,4 -0,75 |
| Schicht 2 | 1,2 | 0,2 -0,4 |
| Schicht 3 | 5,3 | 0,75-1 |
| Schicht 4 | 14,1 | 1 -1,75 |
| Schicht 5 | 66,5 | 1 -2,5 |

Schicht 2 wurde als Ringschicht in der Randzone des Katalysators auf Schicht 3 aufgestreut. Der Durchmesser des Katalysators war 190 cm, die lichte Weite der Ringschicht 180 cm. Dem Reaktor wurden aus der 2. Drahtschicht (29) über Leitung 11 stündlich 13 104 kg gasförmiges Gemisch, das neben Abgas 23 Gew.% Methanol, 0,00009 Gew.% Verunreinigungen, 8,8 Gew.% Wasser und 44,9 Gew.% Luft enthielt, zugeführt. Das Gemisch wurde durch den Katalysator geleitet und bei 700°C und 1,3 bar umgesetzt.

Zur Abkühlung der Reaktionsgase im Reaktor (12) wurden über Leitung (17) stündlich 4710 kg Kesselspeisewasser zugeführt und über Leitung (18) stündlich 4710 kg Wasserdampf (5 bar) als Überschuss, der für eine andere Umsetzung verwendet wurde, abgegeben. Die auf 180°C abgekühlten Reaktionsgase wurden über Leitung (20) dem Absorptionsturm (22) zugeführt. Sie wurden in ihm absorbiert, wobei über Leitung (23) stündlich 199 kg Wasser zugeführt wurden. Der Turm (22) besass einen Kreislauf der Absorptionsflüssigkeit über Leitung (19), Austauscher (7) und Leitung (26). Durch die 76°C warme Absorptionslösungen in Leitung (19) wurde die Kreislaufflüssigkeit der Füllkörperkolonne erwärmt. Über Leitung (28) wurde das Abgas abgeführt. Über Leitung (24) wurden stündlich in Form einer 50 gew.%igen wässerigen Formaldehydlösung 2546 kg Formaldehyd (ber. 100%), entsprechend einer Ausbeute von 88,5% der Theorie abgetrennt. Die Lebensdauer des Katalysators betrug 120 Tage. Die Formaldehydlösung hatte einen Gehalt von 2,4 Gew.% Methanol und 0,02 Gew.% Ameisensäure, bezogen auf Formaldehyd (ber. 100%).

*Beispiel 5:* (Vergleich)

(Zeichnung 1)

22 030 kg Luft wurden stündlich durch Leitung (1) der Füllkörperkolonne (2) unterhalb der Oberfläche des Kolonnensumpfes zugeführt. Im Gegenstrom wurden stündlich 17 310 kg eines Rohmethanol-Wasser-Gemisches (1 Gew.% Verunreinigung, 69,6 Gew.% Methanol, 29,4 Gew.% Wasser) über Leitung (3) sowie 310 000 kg Sumpf (Kreislaufflüssigkeit) der Füllkörperkolonne über Leitung (4), Pumpe (5), Leitung (6), Wärmeaustauscher (7), Leitung (8), Kondensator (9) und Leitung (10) geführt. Die Füllkörperkolonne war 12,8 m lang, hatte einen Durchmesser von 300 cm und enthielt 42 m³ Pallringe als Füllkörper (3,5 cm Länge, 3,5 cm Durchmesser). Der Sumpf (Kreislaufflüssigkeit) enthält 4,7 Gew.% Verunreinigung, 28 Gew.% Methanol und 67,3 Gew.% Wasser. Im Wärmeaustauscher (7) wurde die Kreislaufflüssigkeit im Gegenstrom indirekt mit Absorptionsflüssigkeit über Leitung 19 auf eine Temperatur von 68°C erwärmt. Im Kondensator (9) wurde die Kreislaufflüssigkeit indirekt im Wärmeaustausch mit heissen Reaktionsgasen (über Leitung (20)) auf 79°C erhitzt. Die Temperatur am Eingang der Kreislaufflüssigkeit in die Kolonne (2) betrug 78°C, die Sumpftemperatur der Kolonne (2) 62°C. Über Leitung (13) wurden stündlich 1730 kg Kreislaufflüssigkeit abgezogen und im Verdampfer (14) zusammen mit stündlich 3343 kg Wasser verdampft. Über den Sumpfabzug (15) wurden stündlich 480 kg Verdampfersumpf (80 kg Verunreinigungen, 400 kg Wasser) entzogen. Der stündlich verdampfte Anteil (484,5 kg Methanol, 4108 kg Wasser, 0,5 kg Verunreinigung) wurde aus Verdampfer (14) über Leitung (25) der Leitung (11) zugeführt. Der Durchsatz durch die Füllkörperkolonne betrug 0,24 kg Rohmethanol-Wassergemisch und 0,31 kg Luft pro Stunde und cm² Kolonnenquerschnitt. Die zugeführte Luft wurde durch die Füllkörperkolonne (2), wobei sie einen Anteil an Methanol und Wasser strippte, und dann das resultierende Dampf-Luft-Gemisch durch eine Schicht Drahtfilter (29) geführt. Als Schicht wurde eine Packung Drahtgewirke aus nichtrostendem Chrom-Nickelstahl verwendet. In der Schicht betrug der Drahtdurchmesser 0,55 mm, das freie Schichtvolumen 88 Vol.% bezogen auf das Gesamtvolumen der Schicht. Die Strömungsgeschwindigkeit des Gas-Dampf-Gemischs betrug in der Schicht 0,9 m/sec. Bei dem Durchgang des Gas-Dampf-Gemisches durch die Schicht (29) wurde stündlich ein Anteil (6 kg) abgeschieden und in die Füllkörperkolonne (2) über Leitung (16) zurückgeführt.

Man verwendete eine Anlage mit einem senkrechten Rohrreaktor (12), der an seinem Kopf die Zuführung des dampfförmigen Ausgangsgemischs und die Reaktorhaube enthielt. Die Katalysatorschicht (27) lag unterhalb des Reaktorkopfes. Weiter unten folgte eine Kühlzone, die mit den Leitungen (17) und (18) verbunden war.

In den Reaktor wurde ein Katalysator aus Silberkristallen (429 kg) folgender Zusammensetzung eingetragen:

| | Anteil am Katalysator (Gew.%) | Korngrösse mm |
|---|---|---|
| Schicht 1 | 12,9 | 0,4 -0,75 |
| Schicht 2 | 1,2 | 0,2 -0,4 |
| Schicht 3 | 5,3 | 0,75-1 |
| Schicht 4 | 14,1 | 1 -1,75 |
| Schicht 5 | 66,5 | 1 -2,5 |

Schicht 2 wurde als Ringschicht in der Randzone des Katalysators auf Schicht 3 aufgestreut. Der Durchmesser des Katalysators war 310 cm, die lichte Weite der Ringschicht 305 cm. Dem Reaktor wurden aus der Drahtschicht (29) über Leitung 11 stündlich 42 203 kg gasförmiges Gemisch, das 28,55 Gew.% Methanol, 0,22 Gew.% Verunreinigungen, 19,03 Gew.% Wasser und 52,2 Gew.% Luft enthielt, zugeführt. Das Gemisch wurde durch den Katalysator geleitet und bei 700°C und 1,4 bar umgesetzt.

Zur Abkühlung der Reaktionsgase im Reaktor (12) wurden über Leitung 17 stündlich 14 600 kg Kesselspeisewasser zugeführt und über Leitung 18 stündlich 14 600 kg Wasserdampf (16 bar) als Überschuss, der für eine andere Umsetzung verwendet wurde, abgegeben. Die auf 152°C abgekühlten Reaktionsgase wurden über Leitung 20 dem Kondensator (9) und dann über die Leitung (21) dem Absorptionsturm (22) zugeführt. Sie wurden in ihm absorbiert, wobei über Leitung (23) stündlich 2800 kg Wasser zugeführt wurden. Der Turm (22) besass einen Kreislauf der Absorptionsflüssigkeit über Leitung (19), Austauscher (7) und Leitung (26). Durch die heissen Gase im Kondensator (9), die selbst auf 79°C abgekühlt wurden, und die 70°C warme Absorptionslösungen im Austauscher (7) wurde die Kreislaufflüssigkeit der Füllkörperkolonne erwärmt. Über Leitung (28) wurde das Abgas abgeführt. Über Leitung (24) wurden stündlich in Form einer 40 gew.%igen wässerigen Formaldehydlösung 9819 kg Formaldehyd (ber. 100%), entsprechend einer Ausbeute von 86,9% der Theorie abgetrennt. Die Lebensdauer des Katalysators betrug 20 Tage. Die Formaldehydlösung hatte einen Gehalt von 5,8 Gew.% Methanol und 0,1 Gew.% Ameisensäure, bezogen auf Formaldehyd (ber. 100%).

*Beispiel 6:*

(Zeichnung 1)

Es wurde die gleiche Anlage wie in Beispiel 1 verwendet. Über Leitung (13) wurde keine Kreislaufflüssigkeit abgezogen und über Leitung (25) 3343 kg Wasser zusätzlich zugeführt. Die Umsetzung wurde analog Beispiel 1 mit Methanol anstelle von Rohmethanol und ohne Stufe e (kein Durchgang durch die Drahtschichten) duchgeführt. In Form einer 40 gewichtsprozentigen Formaldehydlösung erhält man 10 000 kg pro Stunde Formaldehyd (ber. 100%), entsprechend einer Ausbeute von 88,5% der Theorie. Die Lebensdauer des Katalysators betrug 120 Tage. Die Formaldehydlösung hat einen Gehalt von 3,2 Gew.% Methanol und 0,025 Gew.% Ameisensäure, bezogen auf Formaldehyd (ber. 100%).

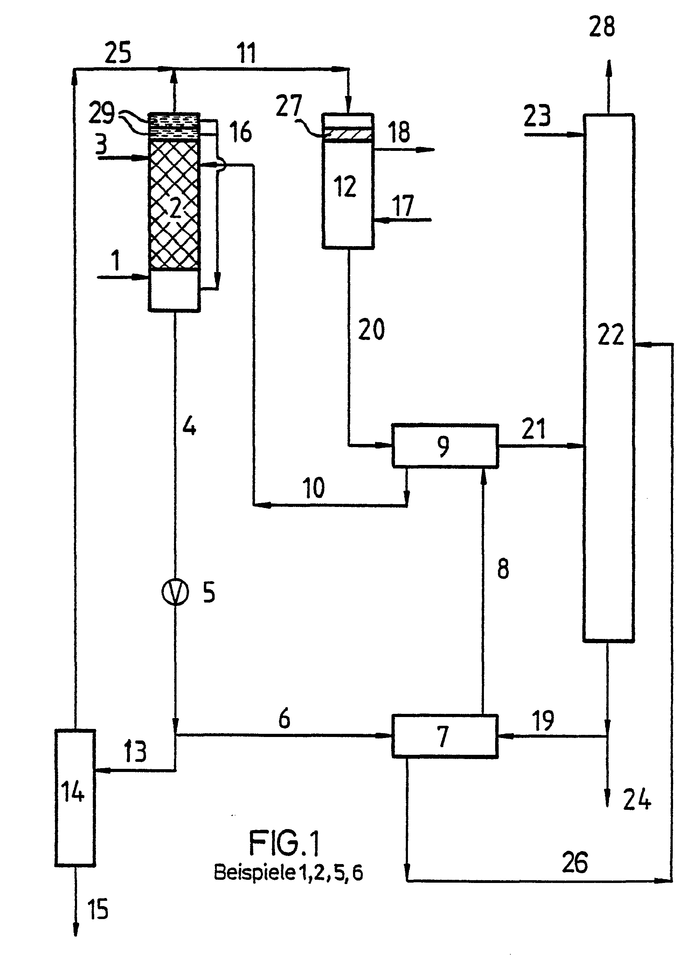

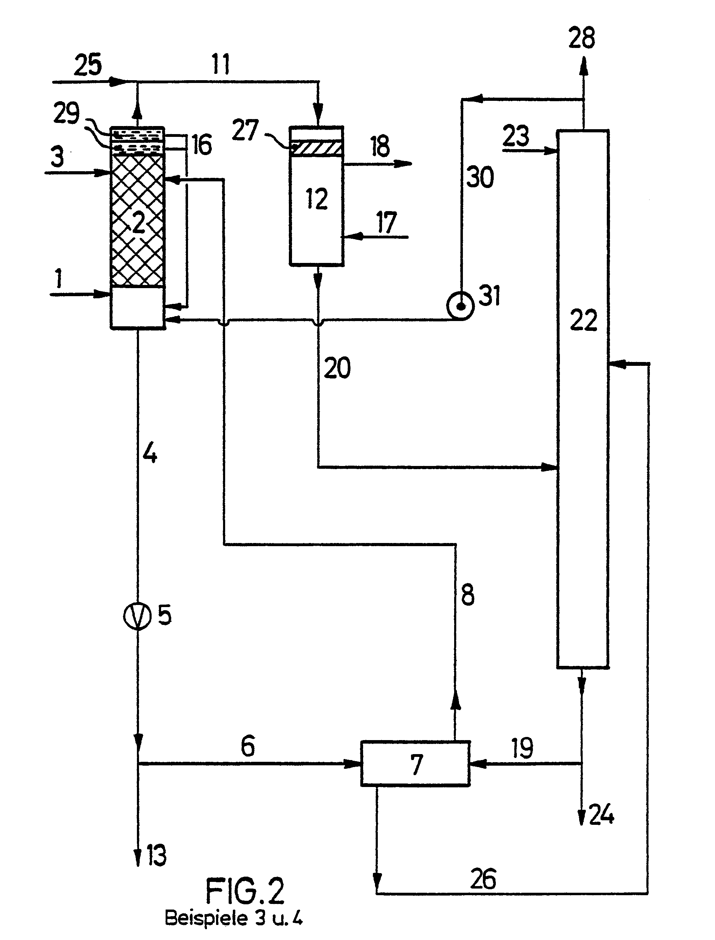

**Patentansprüche**

1. Verfahren zur Herstellung von Formaldehyd durch oxidierende Dehydrierung von Methanol im Gemisch mit Wasser in Gegenwart eines Silberkatalysators bei erhöhter Temperatur und nachfolgender Abkühlung und Absorption der heissen Reaktionsgase, dadurch gekennzeichnet, dass man

a) Methanol und Wasser mit einer Konzentration von 50 bis 90 Gew.% Methanol, bezogen auf das Gesamtgewicht der beiden Stoffe, in eine Füllkörperkolonne, die Füllkörper mit einer Gesamtschichtdicke von mindestens 50 cm und einer Gesamtoberfläche von mindestens 0,5 cm² je cm³ Füllkörperschicht, einen Kreislauf an Flüssigkeit von 15 bis 90 g /min je g/min der Kolonne zugeführtes Methanol, eine Konzentration der Kreislaufflüssigkeit von 10 bis 45 Gew.% Methanol, eine Temperatur am Eingang der Kreislaufflüssigkeit in die Kolonne von 50 bis 86°C und eine von dieser Eingangstemperatur um 10 bis 25°C niedrigere Sumpftemperatur von 40 bis 70°C besitzt, leitet,

b) mit Luft, Inertgas und/oder Abgas einen Anteil an Methanol und Wasser durch Strippen mit einem Durchsatz von 0,5 bis 3 t Methanol und Wasser pro Stunde und m² Kolonnenquerschnitt aus der Füllkörperkolonne abtrennt,

c) das gebildete gasförmige Gemisch von Methanol, Wasser sowie Luft, Inertgas und/oder Abgas mit einer Belastung von 0,5 bis 3 t Methanol je m² Katalysatorbettquerschnitt und Stunde in Gegenwart eines Silberkatalysators bei einer Temperatur von 550 bis 750°C umsetzt, und

d) schliesslich die heissen Reaktionsgase abkühlt und absorbiert, wobei die anfallende Absorptionswärme und gewünschtenfalls Reaktionswärme und/oder Kondensationswärme ganz oder teilweise zur Erhitzung der Kreislaufflüssigkeit verwendet werden.

2. Verfahren nach Anspruch 1, dadurch gekennzeichnet, dass Verunreinigungen enthaltendes Methanol verwendet wird und man die Massnahmen der Stufen

a) und b), dann die Massnahmen einer Stufe

e), worin man das gebildete gasförmige Gemisch von Methanol, Wasser sowie Luft, Inertgas und/oder Abgas durch zwei Schichten Drahtfilter mit einem Drahtdurchmesser von 0,1 bis 0,5 mm und einem freien Schichtvolumen von 90 bis 99,5 Vol.%, bezogen auf das Gesamtvolumen einer Drahtschicht, mit einer Strömungsgeschwindigkeit von 7 bis 13 m/sec in der ersten Schicht und von 1 bis 4 m/sec in der zweiten Schicht, leitet, und schliesslich mit dem gasförmigen Gemisch die Massnahmen der Stufen

c) und d) durchführt.

## Claims

1. A process for the preparation of formaldehyde by oxidative dehydrogenation of methanol as a mixture with water, in the presence of a silver catalyst at elevated temperatures, and subsequent cooling and absorption of the hot reaction gases, wherein

a) a mixture of methanol and water with a concentration of from 50 to 90% by weight, based on the total weight of the two substances, of methanol is passed into a packed column which possesses a packing, having a total layer thickness of not less than 50 cm and a total surface area of not less than 0.5 cm² per cm³ of packing, a liquid circulation of from 15 to 90 g/min per g/min of methanol fed to the column, and a concentration of from 10 to 45% by weight of methanol in the recycle liquid, the temperature at the point of entry of the recycle liquid into the column being from 50 to 86°C and the bottom temperature being 10 to 25°C lower than this entry temperature, i.e. form 40 to 70°C,

b) methanol and water are separated off from the packed column with air, an inert gas and/or off-gas by stripping, the throughput being from 0,5 to 3 tonnes of methanol and water per hour per m² of column cross-section,

c) the resulting gaseous mixture of methanol, water, air, inert gas and/or off-gas is converted, at a space velocity of from 0.5 to 3 tonnes of methanol per m² of catalyst bed cross-section per hour, in the presence of a silver catalyst and at form 550 to 750°C, and

d) finally, the hot reaction gases are cooled and absorbed, the resulting heat of absorption and, if desired, the heat of reaction and/or the heat of condensation being partly or completely used for heating the recycle liquid.

2. A process as claimed in claim 1, wherein methanol containing impurities is used, and the measures of stages

a) and b), then the measures of a stage

e), wherein the resulting gaseous mixture of methanol, water, air, inert gas and/or off-gas is passed through two layers of wire filter having a wire diameter of from 0.1 to 0.5 mm and a free layer volume of from 90 to 99.5 vol.%, based on the total volume of one wire layer, at a flow rate of from 7 to 13 m/sec in the first layer and from 1 to 4 m/sec in the second layer, and finally, using the gaseous mixture, the measures of stages

c) and d) are carried out.

## Revendications

1. Procédé de préparation de formaldéhyde par déshydrogénation par voie d'oxydation de méthanol en mélange avec de l'eau en présence d'un catalyseur à l'argent à température élevée, suivie de refroidissement et d'absorption des gaz chauds de la réaction, caractérisé en ce que:

a) on fait passer le méthanol et l'eau avec une concentration de 50 à 90% en poids de méthanol, sur la base du poids total des deux substances, dans une colonne à garnissage, qui contient des corps de remplissage avec une épaisseur de couche totale d'au moins 50 cm et une surface totale d'au moins 0,5 cm² par cm³ de couche de corps de remplissage, et présente un recyclage de liquide de 15 à 90 g/mn par g/mn de méthanol introduit dans la colonne, une concentration de 10 à 45% en poids de méthanol dans le liquide de recyclage, une température de 50 à 86°C à l'entrée du liquide de recyclage dans la colonne et une température de bas de colonne de 40 à 70°C, inférieure de 10 à 25°C à cette température d'entrée,

b) avec de l'air, un gaz inerte et/ou du gaz résiduaire, on sépare de la colonne à garnissage une fraction de méthanol et d'eau par strippage avec un débit de 0,5 à 3 t de méthanol et d'eau par heure et par m² de section de la colonne,

c) on fait réagir le mélange gazeux formé de méthanol, d'eau, ainsi que d'air, de gaz inerte et/ou de gaz résiduaire avec une charge de 0,5 à 3 t de méthanol par m² de section de lit de catalyseur et par heure, en présence d'un catalyseur à l'argent à une température de 550 à 750°C, et

d) on refroidit et on absorbe enfin les gaz chauds de la réaction, la chaleur d'absorption produite et, au cas où on le désire, la chaleur de réaction et/ou la chaleur de condensation étant utilisées en totalité ou en partie pour réchauffer le liquide de recyclage.

2. Procédé selon la revendication 1, caractérisé en ce que du méthanol contenant des impuretés est utilisé et en ce qu'on exécute les dispositions des phases

a) et b), puis les dispositions d'une phase

e) dans laquelle on fait passer le mélange gazeux formé de méthanol, d'eau, ainsi que d'air, de gaz inerte et/ou de gaz résiduaire à travers deux couches de filtre de fil métallique ayant un diamètre de fil de 0,1 à 0,5 mm et un volume libre de la couche de 90 à 99,5% en volume sur la base du volume total d'une couche de fil métallique, à une vitesse d'écoulement de 7 à 13 m/s dans la première couche et de 1 à 4 m/s dans la seconde couche, et enfin, avec le mélange gazeux, les dispositions des phases c) et d).

0 150 436

FIG.1
Beispiele 1,2,5,6

FIG.2
Beispiele 3 u. 4